# EUROPEAN PATENT APPLICATION

(11) **EP 4 289 439 A2**
(43) Date of publication of application: **13.12.2023**
(21) Application number: 22750085.7
(22) Date of filing: 07.02.2022
(51) Int. Cl.: A61K 35/744, A61P 43/00, A61P 11/00, A61P 1/16, C12N 1/20, A23L 33/135, A23K 10/16

(54) **PHARMACEUTICAL COMPOSITION FOR PREVENTION OR TREATMENT OF FIBROSIS COMPRISING LEUCONOSTOC CITREUM STRAIN AS ACTIVE INGREDIENT**

(30) Priority: 08.02.2021 KR 20210017794; 04.02.2022 KR 20220014809
(71) Applicant: Liscure Biosciences Co., Ltd., Seongnam-si, Gyeonggi-do 13486 (KR)
(72) Inventor: CHIN, Hwa Sup, Yongin-si Gyeonggi-do 16824 (KR)
(74) Representative: Cabinet Becker et Associés
(86) International application number: PCT/KR2022/001869
(87) International publication number: WO 2022/169336

(57) **Abstract**

The present invention relates to a composition comprising a *Leuconostoc citreum* strain or a culture thereof as an active ingredient for preventing, improving or treating fibrosis, specifically pulmonary fibrosis, renal fibrosis or hepatic fibrosis.

According to the present invention, since the composition exhibits excellent preventive and therapeutic effects on various fibrosis, the composition may be usefully used as a composition for the treatment, prevention, improvement, or the like of fibrosis in humans or animals.

## Description

### [Technical Field]

The present invention relates to a composition comprising a *Leuconostoc citreum* strain or a culture thereof as an active ingredient for preventing, improving or treating fibrosis, specifically pulmonary fibrosis, renal fibrosis or hepatic fibrosis.

### [Background Art]

Fibrosis refers to a high degree of deposition of fibrous connective tissues, which is caused by an imbalance between proliferation and degradation of fibrous tissues. A common feature of these diseases is hyperproliferation of fibrous cells, and tissue and organ fibrosis includes pulmonary fibrosis, hepatic fibrosis, chronic pancreatitis, scleroderma, renal glomerular fibrosis, and multiple organ fibrosis caused by radiation chemotherapy and tissue transplantation, etc. Since the fibrosis process progresses chronically, perfect treatment is difficult.

The pulmonary fibrosis is a disease in which inflammation occurs in the lung tissue and fibrosis due to scarring occurs, and a normal oxygen exchange function of the lung is inhibited. Initially, the pulmonary fibrosis starts with alveolitis, in which the alveoli are inflamed, and the alveoli are destroyed, scarred, fibrotic, and then functionally lost, resulting in difficulty in breathing. In addition, when the pulmonary fibrosis occurs, a large load is applied on the right ventricle of the heart that sends blood to the lungs, causing pulmonary arterial hypertension, which eventually develops into right-sided heart failure.

The renal fibrosis is a disease in which fibrosis occurs due to accumulated scars caused by inflammation in the renal tissue, and part of the kidney hardens and its function is lost. The renal fibrosis leads to chronic renal failure, and the chronic renal failure is accompanied by anemia, blood coagulation disorders, hypertension, and various complications and infections of cardiopulmonary and gastrointestinal tract. When the renal function is 15% or less of a normal person, the production of erythropoietin in the kidney decreases, which leads to a decrease in production of red blood cells. In addition, uremia caused when the secretion of urine does not actively occur causes severe anemia by reducing the lifespan of red blood cells. In addition, the onset of uremia increases the probability of systemic infection, which is a major factor in the onset of sepsis.

The hepatic fibrosis may be defined as excessive deposition of the extracellular matrix due to chronic intrahepatic inflammation, and when a chronic liver disease persists due to excessive deposition of such extracellular matrix, eventually, the chronic liver disease progresses to liver cirrhosis due to a change in the intrahepatic structure and a decrease in the number of hepatocytes. Representative cells involved in hepatic fibrosis include hepatic stellate cells, Kupffer cells, endothelial cells, and the like. The hepatic stellate cells are activated as a main production source of producing the extracellular matrix and are involved in increasing the production of various extracellular matrixes including collagen. The Kupffer cells are present in the intrahepatic sinusoidal space, and substances produced from activated Kupffer cells affect surrounding hepatocytes, endothelial cells, and hepatic stellate cells to promote hepatic fibrosis. The endothelial cells play an important role in regulating intrahepatic blood flow, and are also involved in the production of growth factors and extracellular matrixes involved in the proliferation of hepatic stellate cells due to inflammation or hepatic fibrosis.

Currently, there is disclosed a preventive or therapeutic effect of fibrosis of a composition including a compound with a specific structure and derivatives or salts thereof (Korean Patent Publication No. 10-2019-0113639) and a red soybean extract or a fraction thereof (Korean Patent Publication No. 10-2019-0003434) as active ingredients, but there is no description about the preventive or therapeutic effect of fibrosis including a microbial strain as an active ingredient.

Under this background, the present inventors studied to find a substance for preventing and treating fibrosis using lactic acid bacteria with little toxicity and side effects, and as a result, confirmed that a *Leuconostoc citreum* strain or its culture had preventive or therapeutic effects on pulmonary fibrosis, renal fibrosis or hepatic fibrosis, and then completed the present invention.

### [Disclosure]

### [Technical Problem]

An object of the present invention is to provide a pharmaceutical composition for preventing or treating fibrosis including a *Leuconostoc citreum* strain as an active ingredient.

Further, another object of the present invention is to provide a food composition or food additive composition for preventing or improving fibrosis including a *Leuconostoc citreum* strain as an active ingredient.

Further, yet another object of the present invention is to provide a feed composition or feed additive composition for preventing or improving livestock fibrosis including a *Leuconostoc citreum* strain as an active ingredient.

### [Technical Solution]

An aspect of the present invention provides a pharmaceutical composition for preventing or treating fibrosis including a *Leuconostoc citreum* strain or its culture as an active ingredient.

Further, another aspect of the present invention provides a food composition or food additive composition for preventing or improving fibrosis including a *Leuconostoc citreum* strain or its culture as an active ingredient.

Further, yet another aspect of the present invention provides a feed composition or feed additive composition for preventing or improving livestock fibrosis including a *Leuconostoc citreum* strain or its culture as an active ingredient.

### [Advantageous Effects]

According to the present invention, since the *Leuconostoc citreum* strain or its culture exhibits excellent preventive and therapeutic effects on various fibrosis, the *Leuconostoc citreum* strain or its culture may be usefully used as a composition for the treatment, prevention, improvement, or the like of fibrosis in humans or animals.

### [Description of Drawings]

FIG. 1 is a graph showing measurement of the expression level of a related gene α-SMA after treating fibrosis-induced LX-2 cells with a composition according to the present invention.
FIG. 2 is a graph showing measurement of the expression level of a related gene Fibronectin after treating fibrosis-induced LX-2 cells with a composition according to the present invention.
FIG. 3 is a diagram illustrating results of observing the protein amount of α-SMA through Western blot after treating fibrosis-induced LX-2 cells with a *Leuconostoc citreum* WiKim0104 strain.
FIG. 4 is a graph showing measurement of α-SMA after treating a hepatic fibrosis-induced animal model with a *Leuconostoc citreum* WiKim0104 strain.
FIG. 5 is a diagram observing the degree of infiltration and fibrosis of the alveolar tissue after treating a pulmonary fibrosis-induced animal model with a *Leuconostoc citreum* WiKim0104 strain.

### [Best Mode]

A composition including a *Leuconostoc citreum* strain of the present invention or its culture as an active ingredient has a preventive or therapeutic effect of fibrosis, and may be used as a pharmaceutical composition.

The *Leuconostoc citreum* strain of the present invention is a lactic acid bacteria strain. The *Leuconostoc citreum* strain is a probiotic, and has general intestinal regulating and immune enhancing effects of lactic acid bacteria. It is a well-known fact that lactic acid bacteria in *Leuconostoc* sp. have an intestinal regulating effect and an immune enhancing effect.

The *Leuconostoc citreum* strain may be a *Leuconostoc citreum* WiKim0104 strain, and has a nucleic acid sequence represented by SEQ ID NO: 1.

The *Leuconostoc citreum* strain may be inoculated in 0.1 to 10% of a MRS liquid medium and cultured and used at 25 to 37°C for 4 to 48 hours.

The culturing method is preferably a static culture method, but is not limited thereto.

In the present invention, the 'probiotics' is understood as the meaning of 'a living microorganism that has a beneficial effect on the health of the host by improving an intestinal microbial environment of the host in the gastrointestinal tract of animals, including humans'. The probiotics are live microorganisms with probiotic activity, which may have a beneficial effect on the intestinal flora of the host, when fed to humans or animals in the form of dried cells or fermentation products in the form of single or multiple strains.

The fibrosis may be pulmonary fibrosis, renal fibrosis or hepatic fibrosis, but is not limited thereto.

The *Leuconostoc citreum* strain included in the composition according to the present invention may exist as live or dead cells, and may also exist in a dried or freeze-dried form. In addition, a culture of the *Leuconostoc citreum* strain may be an active ingredient, and the culture may include a live cell culture medium or a dead cell supernatant. Types of lactic acid bacteria suitable to be included in various compositions and formulation methods are well known to those skilled in the art.

The composition may be administered orally or parenterally. In the case of parenteral administration, the composition may be administered by intravenous injection, subcutaneous injection, intramuscular injection, intraperitoneal injection, intradermal administration, topical administration, intranasal administration, intrapulmonary administration, intrarectal administration, etc., and preferably intravenous injection, but it is not limited thereto.

A suitable dose of the composition may be variously prescribed by factors, such as a formulation method, an administration type, age, weight, and sex of a patient, a pathological condition, food, an administration time, an administration route, an excretion rate, and response susceptibility.

When the composition of the present invention is used as a pharmaceutical composition, the pharmaceutical composition of the present invention may be prepared by using pharmaceutically suitable and physiologically acceptable adjuvants in addition to the active ingredients. As the adjuvants, excipients, disintegrants, sweeteners, binders, coating agents, expanding agents, lubricants, slip modifiers, flavoring agents, or the like may be used.

The pharmaceutical composition may be preferably formulated by further including at least one kind of pharmaceutically acceptable carrier in addition to the above-described active ingredients for administration.

For example, for formulation in the form of tablets or capsules, the active ingredient may be combined with an oral, a non-toxic pharmaceutically acceptable inert carrier such as ethanol, glycerol, water, or the like. Further, if desired or necessary, suitable binders, lubricants, disintegrants and coloring agents may also be included as a mixture. The suitable binders are not limited thereto, but include natural sugars such as starch, gelatin, glucose or beta-lactose; natural and synthetic gums such as acacia, tragacanth or sodium oleate; sodium stearate, magnesium stearate, sodium benzoate, sodium acetate, sodium chloride and the like. The disintegrant is not limited thereto, but includes starch, methylcellulose, agar, bentonite, xanthan gum, or the like. In the composition formulated with a liquid solution, the pharmaceutically acceptable carrier is suitable for sterilized and living bodies and may use saline, sterilized water, ringer's solution, buffered saline, albumin injection solution, dextrose solution, maltodextrin solution, glycerol, ethanol, and a mixture of at least one of these ingredients, and if necessary, may add other general additives such as antioxidants, buffers, and bacteriostatic agents. In addition, the composition may be prepared in injectable formulations such as aqueous solutions, suspensions, and emulsions, pills, capsules, granules, or tablets by further adding a diluent, a dispersant, a surfactant, a binder, and a lubricant.

Furthermore, as a suitable method in the field, the composition may be formulated preferably according to each disease or ingredient using a method disclosed in Remington's Pharmaceutical Science, Mack Publishing Company, Easton PA.

A composition including a *Leuconostoc citreum* strain of the present invention or its culture as an active ingredient may be used as a food composition or food additive composition for preventing or improving fibrosis.

The food composition may be a health functional food form.

The "functional health food" refers to food prepared and processed using raw materials or ingredients that have functionality useful to the human body in accordance with the Health Functional Food Act (Article 3 (1)), and the "functionality" means regulating nutrients for the structure and function of the human body or obtaining useful effects for health uses such as physiological functions (Article 3 (2)).

The food composition may further include a food additive, and compliance as the "food additive" is determined by the specifications and standards for the corresponding item in accordance with the General Rules of the Korea Food Additives Code and general test methods approved by the Ministry of Food and Drug Safety, unless otherwise specified.

The items disclosed in the "Korea Food Additives Code" may include, for example, chemical composites such as ketones, glycine, potassium citrate, nicotinic acid, and cinnamic acid; natural additives such as persimmon pigment, licorice extract, crystalline cellulose, and guar gum; mixed formulations such as sodium L-glutamate formulations, noodle-added alkali agents, preservative formulations, tar color formulations, etc.

Foods containing the active ingredient of the present invention may include confectionery such as bread, rice cakes, dried confectionery, candy, chocolate, chewing gum, and jam; ice cream products such as ice cream, frozen confectionery, and ice cream powder; dairy products such as milk, low-fat milk, lactose-degraded milk, processed milk, goat milk, fermented milk, butter milk, concentrated milk, milk cream, butter milk, natural cheese, processed cheese, powdered milk, and whey; meat products such as processed meat products, processed egg products, and hamburgers; fish meat products such as fish cake, ham, sausage, bacon, etc.; noodles such as ramen, dried noodles, fresh noodles, fried noodles, deluxe dried noodles, improved soft noodles, frozen noodles, and pasta; drinks such as fruit drinks, vegetable drinks, carbonated drinks, soy milk, lactobacillus drinks such as yogurt, and mixed drinks; seasoned foods such as soy sauce, soybean paste, gochujang, chunjang, cheonggukjang, mixed soy sauce, vinegar, sauces, tomato ketchup, curry, and dressing; margarine, shortening and pizza, but are not limited thereto.

In addition, the composition of the present invention may include various nutrients, vitamins, electrolytes, flavoring agents, coloring agents, pectic acid and salts thereof, alginic acid and salts thereof, organic acid, a protective colloidal thickener, a pH adjusting agent, a stabilizer, a preservative, glycerin, alcohol, a carbonic acid agent used in a carbonated drink, or the like. In addition, the composition of the present invention may include pulps for preparing natural fruit juices, fruit juice beverages or vegetable beverages. These ingredients may be used independently or in combination.

The beverage composition including the active ingredient of the present invention is not particularly limited in other ingredients, and may contain various flavoring agents, natural carbohydrates, or the like as additional ingredients like conventional beverages. Examples of the above-mentioned natural carbohydrates may include general sugars, such as monosaccharides (e.g., glucose, fructose and the like); disaccharides (e.g., maltose, sucrose and the like); and polysaccharides (e.g., dextrin, cyclodextrin and the like), and sugar alcohols such as xylitol, sorbitol, erythritol, and the like. As flavoring agents other than those described above, natural flavoring agents (thaumatin, stevia extract (e.g., Rebaudioside A, glycyrrhizin etc.)) and synthetic flavoring agents (saccharin, aspartame, etc.) may be advantageously used.

Further, a composition including a *Leuconostoc citreum* strain of the present invention or its culture as an active ingredient may be used as a feed composition or feed additive composition for preventing or improving livestock fibrosis.

When the composition is prepared as a feed additive, the composition may be prepared in the form of 20 to 90% high-concentrate or powder or granules. The feed additive may further include any one or one or more of organic acids such as citric acid, fumaric acid, adipic acid, lactic acid, and malic acid, phosphates such as sodium phosphate, potassium phosphate, acidic pyrophosphate, and polyphosphate, or natural antioxidants such as polyphenol, catechin, alpha-tocopherol, rosemary extract, vitamin C, green tea extract, licorice extract, chitosan, tannic acid, phytic acid, and the like. When prepared as a feed, the composition may be formulated in a conventional feed form, and may include general feed ingredients together.

The feed and the feed additives may further include grains, such as milled or ground wheat, oats, barley, corn and rice; vegetable protein feeds, such as feeds based on rape, soybean, and sunflower as a main ingredient; animal protein feeds, such as blood meal, meat meal, bone meal and fish meal; sugar and dairy products, such as dry ingredients consisting of various powdered milk and whey powder, and in addition, nutritional supplements, digestion and absorption enhancers, growth promoters, and the like may be further included.

The feed additive may be administered to animals alone or also administered in combination with other feed additives in an edible carrier. In addition, the feed additives may be easily administered to animals as a top dressing, directly mixed with animal feed, or in an oral formulation separate from feed. When the feed additive is administered separately from animal feed, the feed additive may be prepared as an immediate release or sustained release formulation in combination with a pharmaceutically acceptable edible carrier, as well-known in the art. Such edible carriers may be solids or liquids, for example corn starch, lactose, sucrose, soybean flakes, peanut oil, olive oil, sesame oil and propylene glycol. When the solid carrier is used, the feed additive may be tablets, capsules, powder, troches or sugar-containing tablets or microdispersible top dressing. When the liquid carrier is used, the feed additive may be formulations of gelatin soft capsules, syrups, suspensions, emulsions, or solution formulations.

In addition, the feed and the feed additive may contain auxiliary agents, such as preservatives, stabilizers, wetting agents or emulsifying agents, solution accelerators, and the like. The feed additive may be used to be added to an animal feed by steeping, spraying or mixing.

The feed or feed additive of the present invention may be applied to a plurality of animal diets including mammals, poultry and fish.

The mammals may be used for pigs, cows, horses, sheep, rabbits, goats, rats, hamsters, and guinea pigs, which are rodents and laboratory rodents, as well as pets (e.g., dogs and cats). The poultry may also be used for chickens, turkeys, ducks, geese, pheasants, and quails. The fish may be used for carp, crucian carp, trout, etc., but the present invention is not limited thereto.

### [Modes]

Hereinafter, the present invention will be described in more detail through Examples. These Examples are to explain the present invention in more detail, and it will be apparent to those skilled in the art that the scope of the present invention is not limited by these Examples in accordance with the gist of the present invention.

### Example 1. Culture of strain

A *Leuconostoc citreum* WiKim0104 (accession number KCCM12420P) strain was distributed with the permission of the depositor, Korea Food Research Institute, and experiments were conducted.

The distributed *Leuconostoc citreum* WiKim0104 strain was inoculated at 1% in 30 ml of an MRS liquid medium and static cultured at 30°C for 18 hours. After incubation, the culture solution was stored separately by centrifugation at 3000 rpm for 10 minutes, and the cells were washed three times with a phosphate buffered saline (PBS) solution to remove the remaining medium component.

### Example 2. Measurement of expression levels of related genes in hepatic stellate cells

When LX-2 cells, which were hepatic stellate cells, were plated in a 6-well plate, treated with TGF-β at a concentration of 10 ng/ml in serum free media, and then treated with a *Leuconostoc citreum* WiKim0104 strain, the expression levels of fibrosis-related genes, Fibronectin and α-SMA, were confirmed by qRT-PCR.

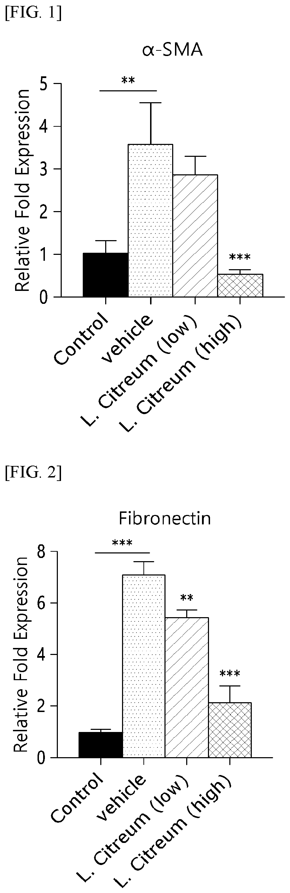

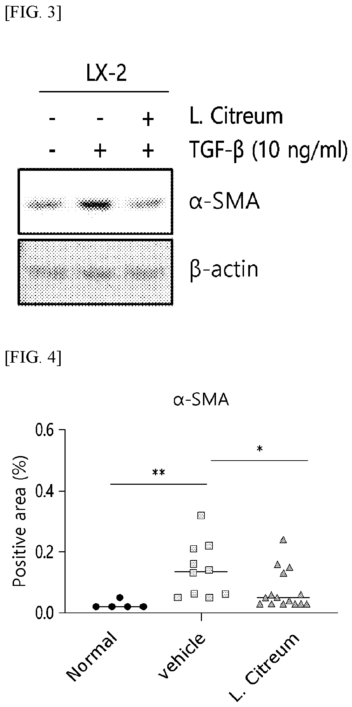

The prepared *Leuconostoc citreum* WiKim0104 strain was treated in a transwell chamber (4 µm pore size) upper chamber at 1 × 10⁷ or 1 × 10⁸ CFU and the LX-2 cells were treated with TGF-β in a lower chamber and cultured for 24 hours. After 24 hours, the LX-2 cells were harvested, RNA was isolated, and then changes in expression levels of fibrosis-related genes were confirmed by qRT-PCR, and the results were illustrated in FIGS. 1 and 2. In addition, as a result of confirming the protein expression level of α-SMA in the LX-2 cells by Western blot, it was confirmed that the protein amount of α-SMA increased by TGF-β significantly decreased in a *Leuconostoc citreum* treated group (FIG. 3).

As illustrated in FIGS. 1 to 3, it was confirmed that the expression levels of α-SMA and fibronectin decreased significantly when the *Leuconostoc citreum* WiKim0104 strain was treated.

### Example 3. Confirmation of fibrosis inhibitory effect in animal model

### 3-1. Experimental animal mouse conditions

As an experimental animal used in the experiment, male 8-week-old C57BL6 mice were supplied, and bred during an experimental period after a one-week stabilization period in an animal breeding room under an SPF environment maintained at a room temperature of 20 ± 2°C and a humidity of 55 ± 15%. As a feed, a general pellet feed without adding antibiotics was supplied, water was allowed to be consumed at any time, and the experiment was performed after adapting to a diet for 1 week. As a litter, a soft litter exclusively for experimental animals was used, a breeding box and a feeder were exchanged twice a week, and all breeding devices used were washed with an automated washing machine and fully adapted and used. For all animals, general symptoms were observed once a day, and the presence or absence of moribund and dead animals was checked twice a day.

### 3-2. Confirmation of effect on hepatic fibrosis in animal model

The animal prepared in Example 3-1 was administered with a choline-deficient amino acid defined high fat diet (CDAHFD) for 12 weeks to induce hepatic fibrosis with non-alcoholic steatohepatitis, and then orally administered with 2 × 10⁹ cfu/200 µl of a *Leuconostoc citreum* WiKim0104 strain composition for 8 weeks. Thereafter, in order to measure the degree of fibrosis of the hepatic tissue, the hepatic tissue was formalin-fixed, and then a paraffin block was prepared and immunostained with anti-α-SMA. The results obtained by quantifying the staining degree of immunostained α-SMA were illustrated in FIG. 4.

As illustrated in FIG. 4, it was confirmed that an anti-α-SMA positive area was significantly reduced in the *Leuconostoc citreum* strain-administered group.

### 3-3. Confirmation of effect on pulmonary fibrosis in animal model

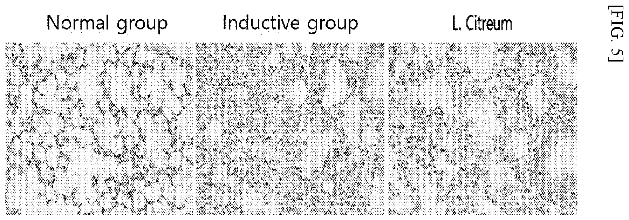

A bile duct ligation model was known to cause pulmonary and renal injury and fibrosis as well as hepatic fibrosis. The bile duct of the mouse prepared in 3-1 was tied twice with a non-absorbable surgical thread. As a control group, the surgery was performed in the same manner without tying the bile duct. After surgery, the mice were grouped according to the Z array method based on changes in body weight, jaundice, and urine color on the day of drug administration. After grouping, the experimental group was orally administered with the *Leuconostoc citreum* WiKim0104 strain at 2 × 10⁹ CFU/day once a day at 0.2 mL/head volume for 2 weeks. The control group ate the same amount of PBS. Thereafter, the lung tissue was excised, washed with physiological saline, and formalin-fixed to prepare a paraffin block. Thereafter, the lung tissue was stained with Hematoxyline-eosin (H&E), and the pathological findings of the lung tissue were observed and the results were shown in FIG. 5.

As illustrated in FIG. 5, in an inductive group, abnormal tissue sites of the alveoli were observed due to infiltration and fibrosis of inflammatory cells, but it was confirmed that the degree thereof was significantly reduced in the *Leuconostoc citreum* strain administered group.

As the result, it may be confirmed that the composition including the *Leuconostoc citreum* WiKim0104 strain or its culture as an active ingredient according to the present invention has excellent preventive and therapeutic effects on fibrosis such as pulmonary fibrosis, renal fibrosis, hepatic fibrosis, or the like.

### [Accession number]

Depositary Authority Name: Korean Culture Center of Microorganisms (Overseas)
Accession number: KCCM12420P
Accession Date: 20181214

<<Translation>>
Budapest Treaty on the International Recognition of the Deposit of Microorganisms for the Purposes of Patent Procedure International Style
To. Korea Food Research Institute
553 65, Nongsaengmyeong-ro 245
Iseo-myeon, Wanju-gun, Jeollabuk-do, South Korea
Issued pursuant to Rule 7.1 by following international deposit authorities

| | I. IDENTIFICATION OF THE MICROORGANISM | | |
|---|---|---|---|
| IDENTIFICATION FOR DISTINGUISH GIVEN BY DEPOSITOR: Leuconostoc citreum WiKim0104 | | | ACCESSION NUMBER GIVEN BY INTERNATIONAL DEPOSITARY INSTITUTION: KCCM 12420P |

| | II. SCIENTIFIC DESCRIPTION AND/OR PROPOSED TAXONOMIC DESIGNATION | | |
|---|---|---|---|
| MICROORGANISM DESIGNATED IN I INCLUDES DOCUMENTS HAVING FOLLOIIING ITE MS | | | |
| | □ SCIENTIFIC PROPERTY | □PROPOSED TAXONOMIC DESIGNATION | |

| | III. RECEIPT AND DEPOSIT | | |
|---|---|---|---|
| INTERNATIONAL DEPOSITARY INSTITUTION DEPOSITS MICROORGANISM ON I RECEIV ED ON DECEMBER 14, 2018. | | | |

| | IV. RECEIPT OF CONVERSION REQUEST | | |
|---|---|---|---|
| The microorganism identified in I above was received by the International Depositary Authority (on the date of initial deposit) and a request was received to convert the initial deposit into a deposit under the Budapest Treaty (on the date the conversation was received). | | | |

| | V. INTERNATIONAL DEPOSITARY INSTITUTION | | |
|---|---|---|---|
| NAME: KOREA MICROBLAL CONSERVATIO N CENTER. | | SIGNATURE: YOUR. SIGNATURE IN YOUR OWN HANDWRITING | |
| ADDRESS: YURIM BUILDING, HONGJENAE 2GA-GIL, SEODAEMUN-GU, SEOUL, 03641, REPUBLIC OF KOREA | | DATE. December 14, 2018 | |

TRANSLATION IS TRUE TO THE ORIGINAL SU INTELLECTUAL PROPERTY

## Claims

1. A pharmaceutical composition for preventing or treating fibrosis comprising a *Leuconostoc citreum* strain or its culture as an active ingredient.

2. The pharmaceutical composition for preventing or treating fibrosis of claim 1, wherein the *Leuconostoc citreum* strain is a *Leuconostoc citreum* WiKim0104 (accession number KCCM12420P) strain having a nucleic acid sequence represented by SEQ ID NO: 1.

3. The pharmaceutical composition for preventing or treating fibrosis of claim 1, wherein the fibrosis is pulmonary fibrosis, renal fibrosis or hepatic fibrosis.

4. The pharmaceutical composition for preventing or treating fibrosis of claim 1, wherein the composition is able to be administered by oral administration or parenteral administration.

5. The pharmaceutical composition for preventing or treating fibrosis of claim 4, wherein the parenteral administration method is intravenous injection, subcutaneous injection, intramuscular injection, intraperitoneal injection, intradermal administration, topical administration, intranasal administration, intrapulmonary administration or intrarectal administration.

6. A food composition for preventing or improving fibrosis comprising a *Leuconostoc citreum* strain or its culture as an active ingredient.

7. The food composition for preventing or improving fibrosis of claim 6, wherein the food is a health functional food.

8. The food composition for preventing or improving fibrosis of claim 6, wherein the food is any one food selected from the group consisting of bread, rice cake, candy, chocolate, gum, ice cream, milk, cheese, processed meat products, processed fish products, kimchi, soy sauce, soybean paste, gochujang, chunjang, cheonggukjang, vinegar, ketchup, curry, dressing, beverages and fermented milk.

9. A food additive composition for preventing or improving fibrosis comprising a *Leuconostoc citreum* strain or its culture as an active ingredient.

10. A feed composition for preventing or improving livestock fibrosis comprising a *Leuconostoc citreum* strain or its culture as an active ingredient.

11. The feed composition for preventing or improving livestock fibrosis of claim 10, wherein the livestock is any one selected from the group consisting of pigs, cows, horses, sheep, rabbits, goats, mice, hamsters, guinea pigs, dogs, cats, chickens, turkeys, ducks, geese, pheasants, quails, carp, crucian carp and trout.

12. A feed additive composition for preventing or improving livestock fibrosis comprising a *Leuconostoc citreum* strain or its culture as an active ingredient.
